# FASCICULE DE BREVET EUROPEEN

(11) **EP 1 297 800 B1**
(45) Date de publication et mention de la délivrance du brevet: **28.09.2016**
(21) Numéro de dépôt: 02292064.9
(22) Date de dépôt: 21.08.2002
(51) Int. Cl.: A61F 2/46, A61B 17/86, A61F 2/34, A61F 2/30

(54) **Cotyle prothetique de la hanche**
Hüftgelenkpfannenprothese
Acetabular cup hip prosthesis

(30) Priorité: 20.09.2001 FR 0112240
(43) Date de publication de la demande: 02.04.2003
(73) Titulaire: Société Civile Smile-Fit, 68200 Mulhouse (FR); Zimmer France SAS, 25461 Etupes Cedex (FR)
(72) Inventeur: Denis, Christophe, Cabinet Ballot -, 25000 Besancon (FR); Jacobzone, Denis, Cabinet Ballot -, 25000 Besancon (FR); Gagna, Gilles, Cabinet Ballot -, 25000 Besancon (FR); Catimel, Christophe, Cabinet Ballot -, 25000 Besancon (FR); Casanova, Georges, Cabinet Ballot -, 25000 Besancon (FR); Laurent, Marc, Cabinet Ballot -, 25000 Besancon (FR); Didailler, Philippe, Cabinet Ballot -, 25000 Besancon (FR); Cayrol, Michel, Cabinet Ballot -, 25000 Besancon (FR); Fabre-Aubrespy, Loic, Cabinet Ballot -, 25000 Besancon (FR); Le Reun, Dominique, Cabinet Ballot -, 25000 Besancon (FR); Viale, Patrick, Cabinet Ballot -, 25000 Besancon (FR); Morin, Olivier, Cabinet Ballot -, 25000 Besancon (FR); Finet, Pierre, Cabinet Ballot -, 25000 Besancon (FR); Bernhard, Laurent, Cabinet Ballot -, 25000 Besancon (FR); Nicolau, Frédéric, Cabinet Ballot -, 25000 Besancon (FR); Rakover, Jean-Patrick, Cabinet Ballot -, 25000 Besancon (FR); Sauvage, Loic, Cabinet Ballot -, 25000 Besancon (FR); Bene, Bruno, Cabinet Ballot -, 25000 Besancon (FR); Bouraly, Jean-Pierre, Cabinet Ballot -, 25000 Besancon (FR)
(74) Mandataire: Mays, Julie

(56) Documents cités:
- EP-A- 0 728 448
- DE-A- 4 007 789
- FR-A- 2 684 544
- FR-A- 2 686 790
- FR-A- 2 719 761
- US-A- 5 725 590

## Description

L'invention concerne un cotyle pour prothèse artificielle de la hanche présentant un insert ou noyau ancrable dans, une cupule métallique ayant une fente longitudinale partant de l'équateur et disposée perpendiculairement à l'équateur, permettant une déformation élastique lors de la pose de la cupule dans une cavité osseuse préparée et pour reposer dans un état précontraint dans la cavité osseuse.

Une telle cupule prothétique est décrite dans la demande de brevet EP-B-0 728 448. La cupule y est impactée dans une cavité osseuse aux dimensions voisines de la cupule extérieure. La déformation de la cupule se réalise au moyen de deux ailettes dépassantes, qui déforment vers l'intérieur la cupule entaillée sur sa moitié. Par l'impaction ultérieure de l'insert, la cupule est amenée à retrouver sa taille d'origine et les ailettes sont ancrées dans l'os. Les ailettes peuvent dépasser d'une surface de plus du double de l'épaisseur de la cupule.

Un tel agencement ne donne pas l'assurance que la pression entre l'équateur de la cupule et l'os sera maintenue après que les ailettes soient enfoncées dans l'os à l'aide de l'impaction de l'insert. Au moment du contrôle on constate que les ailettes enfoncées ont déjà détruit des parties osseuses.

Par contre une cupule métallique de faible épaisseur, par exemple inférieure à 4 millimètres, et fendue de moitié, suit sans problème la réformation liée à la grandeur des ailettes. La situation devient délicate, lorsque l'on veut employer sur la surface extérieure de la cupule un revêtement non-métallique, comme par exemple de l'hydroxyapatite, destiné à promouvoir la croissance osseuse. Le risque d'un écaillage local est alors certain.

Le but de la présente invention est d'obtenir le même « press-fit » contrôlable pour toutes les grandeurs de cupules.

La présente invention a pour but de remédier à cet inconvénient et concerne à cet effet, un cotyle prothétique de la hanche, composé d'une cupule hémisphérique acétabulaire métallique dans laquelle s'ancre un insert, la cupule étant pourvue d'une fente courbe formant une portion de longitude de l'hémisphère, contenue dans un plan perpendiculaire à l'équateur permettant d'amortir la pose de la cupule dans un acétabulum préalablement préparé et pour y reposer dans un état pré-contraint, caractérisé en ce que la fente longitudinale de la cupule s'étend vers le pôle sous un angle β≤70° mesuré à partir de l'équateur de la cupule et se prolonge en forme de T depuis sa fin, dans les deux directions, en une fente transversale courbe formant globalement une portion de latitude de l'hémisphère, avec un angle de 20°<α/2<95°, laquelle fente transversale se termine à chaque extrémité par un trou de diamètre d>4 mm, de manière à ce que l'ajustement des paramètres α et β confère à la dite cupule une raideur indépendante de sa taille, donc une élasticité constante, afin de procurer à l'opérateur toujours la même résistance à l'impaction, quelque soit le diamètre de la cupule.

Un autre but de l'invention est de permettre l'ancrage avec « press-fit » de cupules minces et métalliques revêtues d'une couche non-métallique favorisant la croissance osseuse.

Selon une autre caractéristique de l'invention, la cupule acétabulaire externe est pourvue sur sa surface extérieure, d'une couche non métallique destinée à promouvoir la croissance de l'os, d'une épaisseur inférieure à 200 µm, une telle épaisseur associée aux caractéristiques d'élasticité constante de la cupule, quelque soit son diamètre, permettant de réduire les contraintes de cisaillement à l'interface entre la cupule et la couche.

Préférentiellement la couche non métallique contient de l'hydroxyapatite, mais celle ci peut être avantageusement remplacée par une couche métallique plus ou moins poreuse, par exemple du titane poreux.

La géométrie donnée à la cupule et à la couche non métallique présente l'avantage, que seules de faibles contraintes de cisaillement apparaissent à l'interface entre la cupule et la couche. La déformation élastique de la cupule est autant nécessaire à la réduction du périmètre qu'à l'augmentation ultérieure du périmètre, afin de créer la précontrainte dans la cavité osseuse. Il est garanti par la forme courbe en T de la fente transversale, que la cupule présente une raideur suffisante dans la partie polaire ainsi que sur la surface opposée à la fente, afin de soutenir l'inser dans sa future direction principale de chargement. Malgré cela, il est possible de rendre la cupule mince, afin de garantir une épaisseur de l'insert suffisante permettant d'avoir une absence de fluage du polyethylène.

En raison de la relative faible déformation de la cupule, la fente longitudinale peut avoir une largeur inférieure à 5 millimètres et la fente transversale une largeur inférieure à 5 millimètres, afin d'offrir à l'os une surface de contact aussi grande que possible. Lorsque la fente longitudinale est limitée à une largeur de 2 millimètres ou moins, celle ci ne peut être, lors d'une impaction modérée, au plus refermée que de la largeur de la fente. Les contraintes de cisaillement à l'interface entre la cupule et le revêtement non-métallique sont également limitées par la flexion limitée de la cupule à l'équateur.

Par un choix adapté des angles α/2 et β, la raideur de la cupule peut être contrôlée de telle manière, que lors d'une charge de compression « F » exercée dans le plan équatorial et perpendiculairement au plan de la fente longitudinale courbe, la largeur de la fente longitudinale se réduise de 0.5 millimètres sous une charge de compression de 100 Newton + / - 20 Newton. Une impaction dosée de la cupule est possible avec cette raideur prédéfinie. Elle a en outre l'avantage, que l'opérateur sente, lors de l'impaction de la cupule dans une cavité osseuse sous-dimensionnée, et en raison de la raideur fixée de la cupule, si le futur « press-fit », qui apparaît comme contrainte périphérique entre la cavité osseuse et la cupule, lors de l'impaction de l'insert, est suffisant.

Par le fait que selon l'invention toutes les cupules constituant un kit avec des diamètres « D » différents, présentent la même raideur, préfixée par rapport à des forces de compression agissant perpendiculairement au plan de la fente longitudinale, la cupule peut restituer indépendamment de sa taille une contrainte égale à l'insert. Lorque l'opérateur pressent une stabilité de la cupule trop faible, il a la possibilité d'élargir ou non un petit peu la cavité osseuse et d'utiliser la taille suivante de la cupule.

L'insert est fixée par encliquetage dans la cupule, laquelle présente un chanfrein à l'équateur sur lequel repose une avancée de la cupule interne. Par l'impaction de l'insert, celui-ci provoque l'expansion de la cupule et de l'acétabulum qui l'entoure, afin de prendre place dans un retrait plat inversé s'étendant de manière conique.

Cette surface conique a préférentiellement un demi-angle ε≤3° avec l'axe polaire. Le chanfrein d'entrée prévue pour l'avancée de l'insert constitue un demi-angle δ≤35° avec l'axe polaire.

La présente invention concerne également les caractéristiques qui ressortiront au cours de la description qui va suivre, et qui devront être considérées isolément ou selon toutes leurs combinaisons techniques possibles.

Cette description donnée à titre d'exemple non limitatif, fera mieux comprendre comment l'invention peut être réalisée en référence aux dessins annexés sur lesquels :
la figure 1 représente schématiquement, en vue de dessus une cupule conforme à l'invention ;
la figure 2 représente schématiquement, en coupe longitudinale de la cupule selon la ligne II II de la figure 1 ;
la figure 3 est une vue schématique présentant la coupe longitudinale selon la figure 2 dans une cavité osseuse avec un insert montre ;
la figure 4 représente schématiquement à échelle agrandie, une coupe longitudinale dans la direction du plan de la fente longitudinale à travers la cupule selon la ligne IV IV de la figure 1 ;
la figure 5 représente schématiquement à échelle agrandie un détail V de la figure 4 au niveau de l'équateur montrant l'angle de l'encliquetage, et
la figure 6 représente schématiquement, un diagramme dans lequel des forces « F » selon la figure 1, qui ont pour effet une réduction de la largeur de la fente longitudinale de 0.5 millimètre, sont reportées pour différents diamètres « D ».

Dans les figures, les mêmes indications sont employées pour les mêmes fonctions.

Les figures présentent une cupule de prothèse de hanche avec une cupule métallique et hémisphérique 1 dans laquelle peut s'ancrer un insert 2, la-dite cupule ayant une épaisseur « s » inférieure à 4 millimètres et pourvue d'une fente longitudinale 5 partant de son équateur 3 et s'étendant dans un plan 4 perpendiculaire à l'équateur 3, afin de permettre une déformation élastique et une assise en état précontraint lors de la pose dans une cavité osseuse 23 préparée.

Par le fait que la fente longitudinale 5 repérée par rapport à l'équateur de la cupule 1 s'étend de l'équateur 3 avec un angle β≤70° vers le pôle 6, et qu'elle se prolonge horizontalement en forme de T depuis son extrémité dans les deux sens en une fente transversale courbe 5 formant globalement une portion de latitude de l'hémisphère avec un angle de 20°<α/2<95° et que celle-ci se termine des deux côtés par un trou 8 d'un diamètre d>4 millimètres, et que la cupule extérieure 1 est pourvue sur sa surface extérieure d'une couche 9 non-métallique promouvant la croissance de l'os, d'une épaisseur 10 inférieure à 60 µm, les forces de cisaillement à l'interface entre la cupule 1 et la couche 9 sont maintenues à un niveau faible.

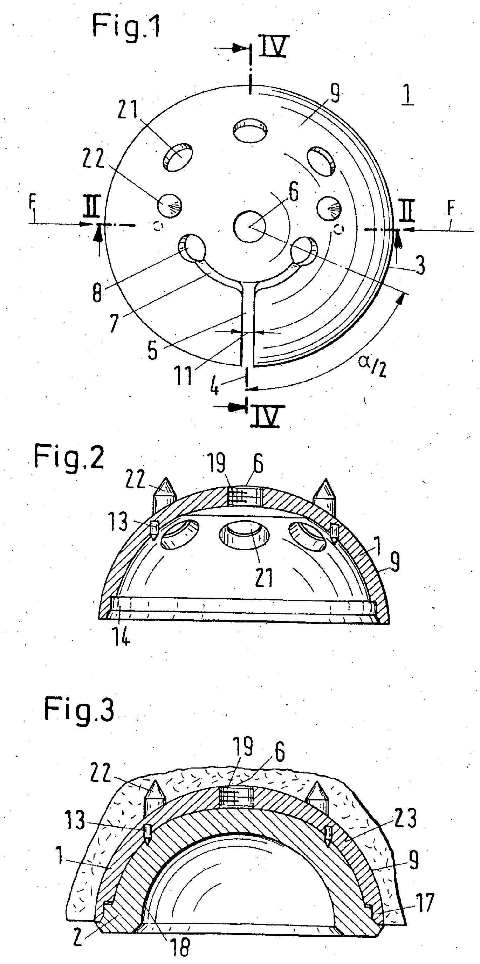

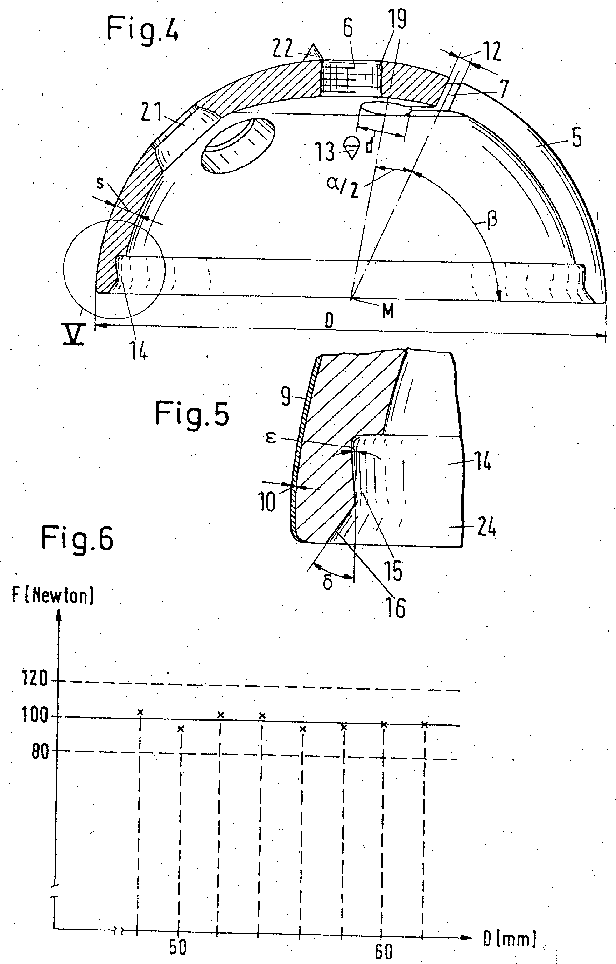

Dans l'exemple de réalisation des figures 1 à 5, la cupule 1 possède une fente longitudinale 5 avec une largeur 11, reposant dans un plan 4, lequel s'étend perpendiculairement à l'équateur 3 et passe par le pôle 6. A la latitude β mesurée par rapport à l'équateur 3 (figure 4), la fente se prolonge dans les deux sens en forme de T en une fente transversale 7 et se termine de chaque côté par un trou 8 d'un diamètre « d » supérieur à 4 millimètres. A son pôle se trouve un filetage 19 pour la fixation d'un instrument d'impaction non représenté. Deux pointes d'ancrages émergentes 22 orientées dans l'axe polaire, constituent une sécurité en rotation de là cupule impactée. Celle-ci peut être ancrée en complément par des vis placées dans des trous de fixation 21, lesquels se trouvent sur la face opposée à la fente longitudinale 5, afin d'influencer au minimum la contre-déformation de la cupule 1 lors de l'impaction de l'insert 2 (figure 2). Dans le cas d'un insert 2 en matière plastique, deux pointes 13 émergent de la cupule 1 et s'enfoncent légèrement dans l'insert 2 afin d'assurer un blocage en rotation. Ces pointes doivent êtres retirées dans le cas d'un insert 2 métallique, et si nécessaire, elles doivent être remplacées par un dispositif de blocage en rotation de forme adaptée.

De par cette géométrie établie de la cupule, il résulte que les plus grandes forces de compression, lors de son impaction dans une cavité osseuse plus petite 23, sont à l'équateur 3, perpendiculaires au plan 4 de la fente longitudinale 5 et à travers l'axe polaire.

Sur la figure 1 est représenté un couple de forces « F » en vis à vis, lequel est utilisé pour la définition de la raideur de la cupule. Les angles α/2 pour la fente transversale 7 et P pour la fente longitudinale 5, sont ajustés pour chaque diamètre de cupule, de telle manière qu'en exerçant en laboratoire un couple de forces F, la largeur 11 de la fente longitudinale 5 à l'équateur se réduise de 0.5 millimètre sous l'action du couple de forces F de 100 Newton +/- 20 Newton. Comme la déformation de la largeur de la fente 11 est la plus élevée pour la fente longitudinale 5, la largeur de fente 12 de la fente transversale 7 peut être choisie plus petite, par exemple 2 millimètres pour une largeur 11 de 3 millimètres pour la fente longitudinale 5. Sur la figure 6 sont reportées les diamètres extérieurs « D » en millimètres de cupules 1 de différentes tailles, et à chaque fois sont reportés les couples correspondants de force « F » en Newton, que l'on contrôle à l'aide de l'ajustement des angles α/2 et β. C'est ainsi que les diamètres « D » pourront être compris entre 44 et 68 millimètres, couvrant toute la gamme.

Après l'impaction de la cupule, celle-ci est soumise à une précontrainte fixée, qui ne correspond pas encore à la précontrainte définitive d'un « press-fit ». La contrainte périphérique nécessaire pour le « press-fit » à l'équateur est produite lors de l'impaction de l'insert 2 par une avancée périphérique 17 (figure 3) contre un retrait conique 15 de la cupule (figure 5). Cet encliquetage 14 empêche non seulement l'insert 2 de tomber, mais elle produit les forces nécessaires à un « press-fit » entre la cupule 1 et la cavité osseuse 23. Simultanément, elle permet que l'insert 2 soit plaquée contre le fond relativement rigide de la cupule. Ceci est possible, lorsque le demi-angle δ est inférieur ou égal à 35° et lorsque le retrait conique 15 présente un demi-angle ε inférieur ou égal à 3°. Lors de l'impaction de l'insert apparaît un maximum de contrainte, qui permet d'amener l'avancée 17 au-delà de la partie la plus étroite entre le chanfrein 24 et le retrait 15.

Comme matière pour la cupule, on prévoit d'utiliser des alliages métalliques élastiques et tolérés par le corps humain comme par exemple des alliages titane-aluminium-niobium comme le « Protasul 100 » de l'entreprise « Sulzer ». L'insert peut être constitué d'un polyéthylène ou d'un métal résistant à l'abrasion.

Sur la cupule en alliage de titane-aluminium-niobium peut être apposée d'abord par projection plasma sous vide une couche intermédiaire de 15 à 90 µm d'épaisseur en titane pur, sur laquelle pour finir, la couche non-métallique 9 favorisant la repousse osseuse est apposée par projection plasma à l'air. La couche 9 peut contenir de l'hydroxyapatite, du phosphate tricalcium (TCP), un mélange d'hydroxyapatite et de phosphate tricalcium, de l'alumine avec un verre sol-gel (base SiO2) ou des matières céramiques.

La couche 9 peut aussi être d'origine métallique avec des épaisseurs inférieures ou égales à 200 µm. Ces revêtements peuvent être du titane poreux.

## Revendications

1. Cotyle prothétique de la hanche, composé d'une cupule hémisphérique acétabulaire métallique (1) dans laquelle s'ancre un insert (2), la cupule étant pourvue d'une fente courbe (5) formant une portion de longitude de l'hémisphère, contenue dans un plan perpendiculaire à l'équateur (3) permettant d'amortir la pose de la cupule (1) dans un acétabulum préalablement préparé et pour y reposer dans un état pré-contraint, **caractérisé en ce que** la fente longitudinale de la cupule externe s'étend vers le pôle (6) sous un angle β≤70° mesuré à partir de l'équateur et se prolonge en forme de T depuis sa fin, dans les deux directions, en une fente transversale courbe formant globalement une portion de latitude de l'hémisphère, avec un angle de 20°<α/2<95°, laquelle fente transversale se termine à chaque extrémité par un trou (8) de diamètre d>4 mm, de manière à ce que l'ajustement des paramètres α et β confère à la dite cupule externe une raideur indépendante de sa taille, donc une élasticité constante, afin de procurer à l'opérateur toujours la même résistance à l'impaction, quelque soit le diamètre de la cupule.

2. Cupule acétabulaire selon la revendication 1 **caractérisée en ce qu'**elle est pourvue sur sa surface extérieure, d'une couche non métallique (9) destinée à promouvoir la croissance de l'os, d'une épaisseur (10) inférieure à 200 µm, une telle épaisseur associée aux caractéristiques d'élasticité constante de la cupule, quelque soit son diamètre, permettant de réduire les contraintes de cisaillement à l'interface entre la cupule (1) et la couche (9).

3. Cupule acétabulaire externe selon la revendication 2 **caractérisée en ce que** la couche non métallique contient de l'hydroxyapatite.

4. Cupule acétabulaire selon l'une des revendications 1 à 3 **caractérisée en ce que** la fente longitudinale présente une largeur (11) inférieure à 5 millimètres au repos et **en ce que** la fente transversale (7) présente une largeur (12) inférieure à 5 millimètres de manière à conférer à ladite cupule une raideur telle qu'une contrainte de compression F de 100N +/- 20N exercée dans le plan équatorial et perpendiculaire au plan de la fente longitudinale et passant par le centre M, réduise la largeur (11) de la fente longitudinale (5) de 0.5 millimètres.

5. Cupule acétabulaire selon la revendication 4 **caractérisée en ce que** la fente longitudinale a une largeur limitée à 5 millimètres ou moins au repos afin d'éviter de grosses déformations lors de la mise en charge.

6. Cupule acétabulaire selon l'une des revendications 1 à 5, **caractérisée en ce que** le maintien de l'insert se fait par un encliquetage (14) dont le retrait conique (15) dans la cupule (1) correspond à un angle ε<=3°.

7. Cupule acétabulaire selon la revendication 6 **caractérisée en ce que** le chanfrein (16) pour une avancée (17) de l'insert (2) dans la cupule (1) a un angle δ≤35°.

## Patentansprüche

1. Prothetische Hüftgelenkspfanne, bestehend aus einer halbkugelförmigen metallischen Hüftpfannenschale (1), in der ein Einsatz (2) verankert ist, wobei die Pfanne mit einem gebogenen Schlitz (5) versehen ist, der einen Längsteil der Halbkugel bildet, enthalten in einer Ebene senkrecht zum Äquator (3), wodurch die Abfederung der Platzierung der Pfanne (1) in einem zuvor vorbereiteten Acetabulum ermöglicht wird und um dort in einem vorgespannten Zustand zu liegen, **dadurch gekennzeichnet, dass** sich der längliche Schlitz der Außenpfanne in Richtung des Pols (6) unter einem Winkel β≤70° gemessen ab dem Äquator erstreckt und sich in beide Richtungen bis zu seinem Ende in T-Form in die Länge zieht, in einen querlaufenden gebogenen Schlitz, der in seiner Gesamtheit einen Breitenteil der Halbkugel bildet, mit einem Winkel von 20°<α/2<95°, wobei der querlaufende Schlitz an jedem Ende mit einem Loch (8) mit einem Durchmesser d>4 mm endet, sodass die Anpassung der Parameter α und β der Außenpfanne eine Steifheit unabhängig von ihrer Größe verleiht, somit eine konstante Elastizität, um den Bediener stets den gleichen Impaktionswiderstand zu bieten, unabhängig vom Durchmesser der Pfanne.

2. Hüftpfannenschale nach Anspruch 1, **dadurch gekennzeichnet, dass** sie auf ihrer Außenoberfläche mit einer nichtmetallischen Schicht (9) versehen ist, die dazu dient, das Knochenwachstum zu fördern, mit einer Dicke (10) von weniger als 200 µm, wobei eine solche Dicke mit den Eigenschaften der konstanten Elastizität der Pfanne assoziiert wird, unabhängig von ihrem Durchmesser, wodurch ermöglicht wird, die Scherspannungen an der Schnittstelle zwischen der Pfanne (1) und der Schicht (9) zu reduzieren.

3. Außenhüftpfannenschale nach Anspruch 2, **dadurch gekennzeichnet, dass** die nichtmetallische Schicht Hydroxylapatit enthält.

4. Hüftpfannenschale nach einem der Ansprüche 1 bis 3, **dadurch gekennzeichnet, dass** der längliche Schlitz im Ruhezustand eine Breite (11) von weniger als 5 Millimetern aufweist und dass der querlaufende Schlitz (7) eine Breite (12) von weniger als 5 Millimetern aufweist, um der Pfanne eine Steifheit zu verleihen, sodass eine Druckspannung F von 100N +/- 20N, ausgeübt in der Äquatorebene und senkrecht zur Ebene des länglichen Schlitzes und die Mitte M durchlaufend, die Breite (11) des länglichen Schlitzes (5) um 0,5 Millimeter reduziert.

5. Hüftpfannenschale nach Anspruch 4, **dadurch gekennzeichnet, dass** der längliche Schlitz im Ruhezustand eine auf 5 Millimeter oder weniger beschränkte Breite aufweist, um bei der Belastung grobe Deformationen zu vermeiden.

6. Hüftpfannenschale nach einem der Ansprüche 1 bis 5, **dadurch gekennzeichnet, dass** die Haltung des Einsatzes durch eine Rastung (14) erfolgt, deren konischer Einzug (15) in die Pfanne (1) einem Winkel ε<=3° entspricht.

7. Hüftpfannenschale nach Anspruch 6, **dadurch gekennzeichnet, dass** die Fase (16) für einen Vorsprung (17) des Einsatzes (2) in der Pfanne (1) einen Winkel δ≤35° aufweist.

## Claims

1. Acetabular hip prosthesis consisting of a metallic hemispherical acetabular cup (1) into which an insert (2) is anchored wherein the cup is provided with an arcuate slot (5) forming a longitudinal portion of the hemisphere, contained in a plane perpendicular to the equator (3) to cushion the application of the cup (1) into a previously prepared acetabulum and to remain there in a prestressed state, **characterised in that** the longitudinal slot of the external cup extends towards the pole (6) at an angle β≤70° measured from the equator and is continued in the shape of a T to its end in both directions in a transverse curved slot forming globally a portion of the latitude of the hemisphere at an angle of 20°<α/2<95°, wherein said transverse slot is terminated at each end by a hole (8) with a diameter of d>4 mm in such a way that, by adjusting the parameters of α and β, the said external cup is provided with a stiffness independent of its size, i.e. constant elasticity, so that the user is always provided with the same resistance to impaction regardless of the diameter of the cup.

2. Acetabular cup according to claim 1 **characterised in that**, on its external surface, it is provided with a non-metallic layer (9) to allow the bone to grow and with a thickness (10) less than 200 µm wherein said thickness is related to the constant elasticity characteristics of the cup regardless of its diameter in order to reduce the shear stresses at the interface between the cup (1) and the layer (9).

3. External acetabular cup according to claim 2 **characterised in that** the non-metallic layer contains hydroxyapatite.

4. Acetabular cup according to any one of claims 1 to 3 **characterised in that**, when at rest, the width (11) of the longitudinal slot is less than 5 millimetres and that the width (12) of the transverse slot (7) is less than 5 millimetres such that the said cup is provided with a radius such that, when a compressive stress F of 100N +/- 20N is applied in the equatorial plane and perpendicular to the plane of the longitudinal slot and passing through the centre M, the width (11) of the longitudinal slot (5) is reduced by 0.5 millimetres.

5. Acetabular cup according to claim 4 **characterised in that** the width of the longitudinal slot is limited to 5 millimetres or less when at rest in order to avoid large deformations when bearing weight.

6. Acetabular cup according to any one of claims 1 to 5, **characterised in that** the insert is held in place by a locking mechanism (14) wherein the angle ε of the conical taper (15) in the cup (1) is ε<=3°.

7. Acetabular cup according to claim 6 **characterised in that** the angle δ of the chamfer (16) acting as a lead-in (17) for guiding the insert (2) into the cup (1) is α≤35°.
